# EUROPEAN PATENT APPLICATION

(11) **EP 4 356 897 A1**
(43) Date of publication of application: **24.04.2024**
(21) Application number: 22824935.5
(22) Date of filing: 10.06.2022
(51) Int. Cl.: A61K 8/25, A61K 8/02, A61Q 1/00, A61Q 1/10

(54) **POWDER COSMETIC AND PRODUCTION METHOD FOR SAME**

(30) Priority: 14.06.2021 JP 2021098951
(71) Applicant: Shiseido Company, Ltd., Chuo-ku Tokyo 104-0061 (JP)
(72) Inventor: ITO, Shun, Tokyo 104-0061 (JP)
(74) Representative: Cabinet Beau de Loménie
(86) International application number: PCT/JP2022/023514
(87) International publication number: WO 2022/264945

(57) **Abstract**

There is provided a powder cosmetic having improved overall usability and improved moldability and impact resistance when formed into a solid powder cosmetic.

A cosmetic, which is a powder cosmetic comprising 80 to 99% by mass of a powder component and 1 to 20% by mass of an oily component, wherein the powder component comprises a component (A) below and at least one of a component (B) and a component (C) below: component (A): hollow silica particles, having an average particle size of 1 to 10 µm, component (B): solid silica particles, having an average particle size of 2 to 7 µm, and component (C): porous silica particles, having an average particle size of 1 to 30 µm.

## Description

### FIELD

The present invention relates to a powder cosmetic and a method for manufacturing the same.

### BACKGROUND

In recent years, various powder cosmetics such as powder foundation, eye shadow, face powder, and blusher have been developed. Such powder cosmetics often contain particles of, for example, silica.

More specifically, for example, PTL 1 discloses a solid powder cosmetic comprising 1 to 20% by mass of an oily component and hollow nanoparticles composed of silica shells for the purpose of providing a solid powder cosmetic having excellent drop impact resistance while maintaining a satisfactory usability as a cosmetic.

PTL 2 discloses a cosmetic blended with predetermined silica-based particles in the range of 0.1 to 30% by weight. For the silica particles according to PTL 2, the average particle size is in the range of 0.1 to 200 µm, a cavity is present in the inner portion of an outer-shell silica layer, the porosity of the cavity is in the range of 20 to 95% by weight, the outer-shell silica layer is non-porous, and the inner portion of the cavity is under negative pressure.

### [CITATION LIST]

### [PATENT LITERATURE]

[PTL 1] Japanese Unexamined Patent Publication (Kokai) No. 2013-129622
[PTL 2] Japanese Unexamined Patent Publication (Kokai) No. 2011-256098

### SUMMARY

### [TECHNICAL PROBLEM]

In conventional powder cosmetics containing silica particles, it cannot be said that overall usability (i.e., usability derived from a comprehensive tactile feel consisting of a silky feel, a soft feel, and a non-powdery, smooth feel), and moldability and impact resistance when formed into solid powder cosmetics are sufficient, and there is room for further improvement.

The present invention attempts to improve upon the above circumstances and has an object of providing a powder cosmetic having improved overall usability and improved moldability and impact resistance when formed into a solid powder cosmetic.

### [SOLUTION TO PROBLEM]

The present invention for achieving the above object is as follows.

### <Aspect 1>

A cosmetic, which is a powder cosmetic comprising 80 to 99% by mass of a powder component and 1.0 to 20% by mass of an oily component, wherein
the powder component comprises a component (A) below and at least one of a component (B) and a component (C) below:
component (A): hollow silica particles, having an average particle size of 1 to 10 µm,
component (B): solid silica particles, having an average particle size of 2 to 7 µm, and
component (C): porous silica particles, having an average particle size of 1 to 30 µm.

### <Aspect 2>

The cosmetic according to Aspect 1, wherein the powder component comprises the component (A), the component (B), and the component (C).

### <Aspect 3>

The cosmetic according to Aspect 1 or 2, which is a solid powder cosmetic.

### <Aspect 4>

A method for manufacturing the cosmetic according to any one of Aspects 1 to 3, comprising:
dispersing the powder component and the oily component in a solvent to prepare a slurry;
filling the slurry into a container; and
removing the solvent from the slurry after container filling.

### <Aspect 5>

A method for manufacturing the cosmetic according to any one of Aspects 1 to 3, comprising:
dispersing the powder component and the oily component in a solvent to prepare a slurry; and
drying the slurry to obtain a dry powder.

### [ADVANTAGEOUS EFFECTS OF INVENTION]

According to the present invention, a powder cosmetic having improved overall usability and improved moldability and impact resistance when formed into a solid powder cosmetic can be provided.

### DESCRIPTION OF EMBODIMENTS

Hereinafter, embodiments of the present invention will be described in detail. The present invention is not limited to the following embodiments, and various modifications can be made thereto within the scope of the invention.

### <<Powder cosmetic>>

The powder cosmetic of the present invention (hereinafter, also referred to as "the cosmetic of the present invention") is a powder cosmetic comprising 80 to 99% by mass of a powder component and 1 to 20% by mass of an oily component, wherein
the powder component comprises the following component (A) and at least one of the following component (B) and component (C):
component (A): hollow silica particles, having an average particle size of 1 to 10 µm,
component (B): solid silica particles, having an average particle size of 2 to 7 µm, and
component (C): porous silica particles, having an average particle size of 1 to 30 µm.

### <Powder component>

The cosmetic of the present invention comprises a powder component. The content of the powder component of the cosmetic of the present invention relative to the total amount of the cosmetic may be 80% by mass or greater, 85% by mass or greater, 90% by mass or greater, or 95% by mass or greater, and may be 99% by mass or less.

The present inventors have discovered through intensive research that the effect of the present invention can be achieved by using two or more types of specific silica particles in combination, and the effect of the present invention can be further exhibited by using three or more types of specific silica particles in combination.

More specifically, in the present invention, the cosmetic of the present invention comprises the following component (A) and at least one of the following component (B) and component (C) as the powder component, and preferably comprises the following component (A), the following component (B), and the following component (C) from the viewpoint of further exhibiting the effect of the present invention:
component (A): hollow silica particles, having an average particle size of 1 to 10 µm,
component (B): solid silica particles, having an average particle size of 2 to 7 µm, and
component (C): porous silica particles, having an average particle size of 1 to 30 µm.

### (Component (A))

In the present invention, the component (A) refers to hollow silica particles having an average particle size of 1 to 10 µm. Particularly, such hollow silica particles have an effect of imparting the powder cosmetic with a lightweight feel and a smooth feel. In the present invention, the hollow silica particles refer to silica particles having silica as an outer shell and a cavity as an inner core.

The shape of the hollow silica particles is not particularly limited as long as the particles are hollow particles, and may be, for example, spherical, cubic, rugby ball-like, rod-like, spindle-like, columnar, tubular, sheet-like, or a combination thereof. Among these, a spherical shape is preferable because the shape allows the particles to roll on the skin during application, thereby particularly improving the lightweight feel.

In the present invention, the average particle size of the hollow silica particles as the component (A) may be 1 µm or more, 2 µm or more, 3 µm or more, or 4 µm or more, and may be 10 µm or less, 8 µm or less, 6 µm or less, or 4 µm or less.

In the present invention, the average particle size of the silica particles can be determined as a volume-equivalent average particle size measured by a laser light scattering method.

In the present invention, the porosity of the hollow silica particles is not particularly limited, and may be, for example, 20% or greater, 30% or greater, 50% or greater, or 80% or greater, and may be 95% or less or 90% or less.

In the present invention, the oil absorption amount of the hollow silica particles is not particularly limited, and may be, for example, 10 ml/100 g or more, 30 ml/100 g or more, or 50 ml/100 g or more, and may be 100 ml/100 g or less or 80 ml/100 g or less.

Regarding the oil absorption amount of the silica particles in the present invention, silica particles are kneaded with a metal spatula while a given amount of squalane (oil content) is added dropwise, until it can be visually confirmed that all the silica particles are integrated and the oil content is the continuous phase, and the amount of dropped oil at that point is defined as the oil absorption amount.

In the present invention, the hollow silica particles may not be subjected to a surface treatment, or may be subject to a surface treatment. Examples of the surface treatment of the hollow silica particles include, but are not limited to, an amino acid treatment, a silicone treatment, or a metal soap treatment.

The content of the component (A) of the cosmetic of the present invention relative to the total amount of the cosmetic may be, for example, 3.0% by mass or greater, 4.0% by mass or greater, 5.0% by mass or greater, 6.0% by mass or greater, or 7.0% by mass or greater, and may be 20% by mass or less, 15% by mass or less, 10% by mass or less, 8.0% by mass or less, or 6.0% by mass or less.

### (Component (B))

In the present invention, the component (B) refers to solid silica particles having an average particle size of 2 to 7 µm. Particularly, such solid silica particles together with the oily component have an effect of imparting the powder cosmetic with a soft feel. In the present invention, the solid silica particles refer to silica particles not having a cavity in the inner portion thereof.

The shape of the solid silica particles is not particularly limited as long as the particles do not have a cavity in the inner portion thereof, and may be, for example, spherical, cubic, rugby ball-like, rod-like, spindle-like, columnar, tubular, sheet-like, or a combination thereof. Among these, a spherical shape is preferable, for example, from the viewpoint of satisfactory usability derived from the shape thereof during application.

In the present invention, the average particle size of the solid silica particles as the component (B) may be 2 µm or more, 4 µm or more, or 6 µm or more, and may be 10 µm or less, 8 µm or less, or 6 µm or less.

In the present invention, the oil absorption amount of the solid silica particles is not particularly limited, and may be, for example, 10 ml/100 g or more, 15 ml/100 g or more, or 20 ml/100 g or more, and may be 50 ml/100 g or less or 30 ml/100 g or less.

In the present invention, the solid silica particles may not be subjected to a surface treatment, or may be subjected to a surface treatment. Examples of the surface treatment of the solid silica particles include, but are not limited to, an amino acid treatment, a silicone treatment, and a metal soap treatment.

The content of the component (B), when contained in the cosmetic of the present invention, relative to the total amount of the cosmetic may be, for example, 2.0% by mass or greater, 3.0% by mass or greater, 4.0% by mass or greater, or 5.0% by mass or greater, and may be 10% by mass or less, 8.0% by mass or less, or 5.0% by mass or less.

### (Component (C))

In the present invention, the component (C) refers to porous silica particles having an average particle size of 1 to 30 µm. Such porous silica has a relatively high effect of absorbing sebum, and thus has an effect of imparting the powder cosmetic with a particularly silky feel. In the present invention, the porous silica particles refer to silica particles having fine holes on the surface.

The shape of the porous silica particles is not particularly limited as long as the particles are porous, and may be, for example, spherical, cubic, rugby ball-like, rod-like, spindle-like, columnar, tubular, sheet-like, or a combination thereof. Among these, a spherical shape is preferable, for example, from the viewpoint of preventing agglomeration.

In the present invention, the average particle size of the porous silica particles as the component (C) may be 1 µm or more, 3 µm or more, 5 µm or more, or 8 µm or more, and may be 30 µm or less, 20 µm or less, or 10 µm or less.

In the present invention, the oil absorption amount of the porous silica particles is not particularly limited, and may be, for example, 50 ml/100 g or more.

In the present invention, the porous silica particles may not be subjected to a surface treatment, or may be subjected to a surface treatment. Examples of the surface treatment of the porous silica particles include, but are not limited to, an amino acid treatment, a silicone treatment, and a metal soap treatment.

The content of the component (C), when contained in the cosmetic of the present invention, relative to the total amount of the cosmetic may be 2.0% by mass or greater, 3.0% by mass or greater, or 4.0% by mass or greater, and may be 10% by mass or less, 8.0% by mass or less, or 6.0% by mass or less.

### (Relationship between contents of component (A), component (B), and component (C))

When the component (A) and the component (B) are used in combination in the cosmetic of the present invention, the contents thereof may be the same as or different from each other. The content of the component (A) may be larger than the content of the component (B). When the component (A) and the component (C) are used in combination in the cosmetic of the present invention, the contents thereof may be the same as or different from each other. The content of the component (A) may be larger than the content of the component (C). When the component (A), the component (B), and the component (C) are used in combination in the cosmetic of the present invention, the contents thereof may be the same as or different from one another. Among the three, the content of the component (A) may be the largest, the content of the component (C) may be intermediate, and the content of the component (B) may be the smallest. Specifically, when the component (A), the component (B), and the component (C) are used in combination, the content of component (A) : the content of component (B) : the content of component (C) may be, for example, 1.0:0.1 to 2.0:0.1 to 2.0, 1.0:0.2 to 1.5:0.2 to 1.5, 1.0:0.2 to 1.0:0.2 to 1.0, or 1.0:0.3 to 0.8:0.3 to 0.9.

### (Additional powder component)

The cosmetic of the present invention may further comprise one or more additional powder components, in addition to the component (A), the component (B), and the component (C) described above as the powder component. The content of the additional powder component when contained can be appropriately adjusted according to the function or effect thereof or the intended use of the cosmetic.

Examples of the additional powder component include, but are not limited to, mica, talc, kaolin, sericite, muscovite, phlogopite, synthetic mica, synthetic fluorphlogopite, red mica, biotite, calcined talc, calcined sericite, calcined muscovite, calcined phlogopite, boron nitride, vermiculite, magnesium carbonate, calcium carbonate, aluminum silicate, barium silicate, calcium silicate, magnesium silicate, strontium silicate, tungstate metal salts, magnesium, silica, zeolite, barium sulfate, calcined calcium sulfate (calcined gypsum), calcium phosphate, fluorapatite, hydroxyapatite, ceramic powder, metal soaps (for example, zinc myristate, calcium palmitate, and aluminum stearate), photochromic titanium oxide (titanium dioxide sintered with iron oxide), reduced zinc white; organic powders (for example, silicone elastomer powder, silicone powder, silicone resin-coated silicone elastomer powder, polyamide resin powder (nylon powder), polyethylene powder, polystyrene powder, styrene-acrylic acid copolymer resin powder, benzoguanamine resin powder, polytetrafluoroethylene powder, and cellulose powder); inorganic white pigments (for example, titanium dioxide and zinc oxide); inorganic red pigments (for example, iron oxide (Bengala) and iron titanate); inorganic brown pigments (for example, γ-iron oxide); inorganic yellow pigments (for example, yellow iron oxide and ocher); inorganic black pigments (for example, black iron oxide and lower titanium oxide); inorganic purple pigments (for example, mango violet and cobalt violet); inorganic green pigments (for example, chromium oxide, chromium hydroxide, and cobalt titanate); inorganic blue pigments (for example, ultramarine and navy blue); pearl pigments (for example, bismuth oxychloride, fish scale foil, mica titanium, iron oxide-coated titanium mica, low-grade titanium oxide-coated titanium mica, titanium mica having photochromic properties, those using talc, glass, synthetic fluorphlogopite, silica, and bismuth oxychloride as substrates in place of mica, those coated with low-grade titanium oxide, colored titanium oxide, iron oxide, alumina, silica, zirconia, zinc oxide, cobalt oxide, and aluminum as coatings in addition to titanium oxide, those coated with resin particles on the pearl pigment surface as functional pearl pigments (Japanese Unexamined Patent Publication (Kokai) No. 11-92688), those coated with aluminum hydroxide particles on the pearl pigment surface (Japanese Unexamined Patent Publication (Kokai) No. 2002-146238), those coated with zinc oxide particles on the pearl pigment surface (Japanese Unexamined Patent Publication (Kokai) No. 2003-261421)), those coated with barium sulfate particles on the pearl pigment surface (Japanese Unexamined Patent Publication (Kokai) No. 2003-61229); metal powder pigments (for example, aluminum powder and copper powder); organic pigments of zirconium, barium, and aluminum lakes (for example, organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404, Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1); and natural pigments (for example, chlorophyll and β-carotene).

The additional powder component may be subjected to a surface treatment with a higher fatty acid, a metal soap, an oil/fat, a wax, a silicone compound, a fluorine compound, a hydrocarbon, a surfactant, or a dextrin fatty acid ester.

As described above, the cosmetic of the present invention can improve overall usability by comprising the component (A) and at least one of the component (B) and the component (C) described above, and thus may not substantially comprise a resin powder blended for the purpose of imparting a soft feel, as reported in the prior art. The content of such a resin powder as a powder component, when contained in the cosmetic of the present invention, relative to the total amount of the cosmetic may be 5.0% by mass or less, 4.0% by mass or less, 3.0% by mass or less, 2.0% by mass or less, 1.0% by mass or less, 0.5% by mass or less, or 0.1% by mass or less.

### <Oily component>

The cosmetic of the present invention comprises an oily component. The content of the oily component of the cosmetic of the present invention relative to the total amount of the cosmetic may be 1.0% by mass or greater, 5.0% by mass or greater, or 10% by mass or greater, and may be 20% by mass or less or 15% by mass or less.

The oily component is not particularly limited as long as, for example, the component can be generally used in cosmetics. More specifically, examples thereof include, but are not limited to, liquid oils/fats, solid oils/fats, waxes, hydrocarbons, higher fatty acids, higher alcohols, synthetic ester oils, and silicone oils. In the present invention, "oily component" includes oils and oil-soluble components.

In the following description, POE is an abbreviation for polyoxyethylene, and POP for polyoxypropylene. The number in parentheses after POE or POP represents the average number of added moles of POE groups or POP groups in the compound.

Examples of the liquid oils/fats include, but are not limited to, avocado oil, camellia oil, turtle oil, macadamia nut oil, corn oil, mink oil, olive oil, rapeseed oil, egg yolk oil, sesame oil, perilla oil, wheat germ oil, sasanqua oil, castor oil, linseed oil, safflower oil, cottonseed oil, Inca Inchi oil, soybean oil, peanut oil, tea seed oil, kaya oil, rice bran oil, neem oil, Japanese paulownia oil, jojoba oil, germ oil, and triglycerin.

Examples of the solid oils/fats include, but are not limited to, cocoa butter, coconut oil, horse tallow, hydrogenated coconut oil, palm oil, beef tallow, mutton tallow, hydrogenated beef tallow, palm kernel oil, lard, beef bone fat, Japan wax kernel oil, hydrogenated oil, beef leg fat, Japan wax, and hydrogenated castor oil.

Examples of the waxes include, but are not limited to, beeswax, candelilla wax, cotton wax, carnauba wax, bayberry wax, Chinese wax, whale wax, Montan wax, bran wax, lanolin, kapok wax, lanolin acetate, liquid lanolin, sugarcane wax, lanolin fatty acid isopropyl, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin alcohol ether.

Examples of the hydrocarbon oils include, but are not limited to, liquid paraffin, ozokerite, squalene, pristane, paraffin, ceresin, squalene, petroleum jelly, and microcrystalline waxes.

Examples of the higher fatty acids include, but are not limited to, lauric acid, myristic acid, palmitic acid, stearic acid, behenic acid, oleic acid, undecylenic acid, thioctic acid, isostearic acid, linoleic acid, linolenic acid, eicosapentaenoic acid (EPA), and docosahexaenoic acid (DHA).

Examples of the higher alcohols include, but are not limited to, linear chain alcohols (for example, lauryl alcohol, cetyl alcohol, stearyl alcohol, behenyl alcohol, myristyl alcohol, oleyl alcohol, and cetearyl alcohol); and branched chain alcohols (for example, monostearin glyceryl ether (butyl alcohol), 2-decyltetradecanol, lanolin alcohol, cholesterol, phytosterol, hexyldecanol, isostearyl alcohol, and octyldodecanol).

Examples of the synthetic ester oils include, but are not limited to, isopropyl myristate, cetyl octoate, octyldodecyl myristate, isopropyl palmitate, butyl stearate, hexyl laurate, myristyl myristate, decyl oleate, hexyldecyl dimethyl octoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glyceryl di-2-heptylundecanoate, trimethylolpropane tri-2-ethylhexanoate, trimethylolpropane triisostearate, pentaerythritol tetra-2-ethylhexanoate, glyceryl tri-2-ethylhexanoate, glyceryl trioctanoate, glyceryl triisopalmitate, trimethylolpropane triisostearate, cetyl 2-ethylhexanoate, 2-ethylhexyl palmitate, glyceryl trimyristate, tri-2-heptyl undecanoic acid glyceride, castor oil fatty acid methyl ester, oleyl oleate, acetoglyceride, 2-heptylundecyl palmitate, diisobutyl adipate, N-lauroyl-L-glutamate-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, and triethyl citrate.

Examples of the silicone oils include, but are not limited to, silicone compounds such as dimethyl polysiloxane, methyl hydrogen polysiloxane, methyl phenyl polysiloxane, stearyloxy methyl polysiloxane, polyether modified organopolysiloxane, fluoroalkyl-polyoxyalkylene co-modified organopolysiloxane, alkyl modified organopolysiloxane, unterminated modified organopolysiloxane, fluorine modified organopolysiloxane, amino modified organopolysiloxane, silicone gel, acrylic silicone, trimethylsiloxy silicic acid, and silicone RTV rubber.

### <Additional components>

The cosmetic of the present invention can be appropriately blended with additional components, for example, esters, anionic surfactants, cationic surfactants, amphoteric surfactants, nonionic surfactants, humectants, water-soluble polymers, thickeners, film-forming agents, ultraviolet absorbers, sequestrants, lower alcohols, polyhydric alcohols, saccharides, amino acids, organic amines, polymer emulsions, pH adjusters, skin nutrients, vitamins, antioxidants, antioxidant aids, fragrances, and water, as needed, in a range that does not impair the effect of the present invention, and can be manufactured according to conventional methods, depending on the intended formulation.

Specific components that can be blended are listed below. The powder cosmetic can be prepared by blending one or more of the optional components below with the indispensable components above.

Examples of the anionic surfactants include, but are not limited to, fatty acid soaps (for example, sodium laurate and sodium palmitate); higher alkyl sulfate salts (for example, sodium lauryl sulfate and potassium lauryl sulfate); alkyl ether sulfate salts (for example, PE-triethanolamine lauryl sulfate and POE-sodium lauryl sulfate); N-acyl sarcosinates (for example, sodium lauroyl sarcosinate); higher fatty acid amide sulfonates (for example, sodium N-methyl-N-myristoyl taurate, sodium coco methyl tauride, and sodium lauryl methyl tauride); phosphate ester salts (POE-sodium oleyl ether phosphate and POE-stearyl ether phosphate); sulfosuccinates (for example, sodium di-2-ethylhexyl sulfosuccinate, monolauroyl monoethanolamide polyoxyethylene sodium sulfosuccinate, and sodium lauryl polypropylene glycol sulfosuccinate); alkylbenzene sulfonates (for example, sodium linear dodecylbenzene sulfonate, triethanolamine linear dodecylbenzene sulfonate, and linear dodecylbenzene sulfonic acid); higher fatty acid ester sulfate ester salts (for example, hydrogenated coco glycerol sodium sulfate); N-acylglutamates (for example, monosodium N-lauroylglutamate, disodium N-stearoylglutamine, and monosodium N-myristoyl-L-glutamate); sulfated oils (for example, sulfonated castor oil); POE-alkyl ether carboxylic acid; POE-alkyl allyl ether carboxylate; α-olefm sulfonate; higher fatty acid ester sulfonate; secondary alcohol sulfate ester salt; higher fatty acid alkylolamide sulfate ester salt; sodium lauroyl monoethanolamide succinate; N-palmitoyl aspartic acid diethanolamine; and sodium caseinate.

Examples of the cationic surfactants include, but are not limited to, alkyltrimethylammonium salts (for example, stearyltrimethylammonium chloride and lauryltrimethylammonium chloride); alkylpyridinium salts (for example, cetylpyridinium chloride); distearyldimethylammonium chloride dialkyldimethylammonium salt; poly(N,N'-dimethyl-3,5-methylenepiperidinium) chloride; alkyl quaternary ammonium salt; alkyldimethylbenzylammonium salt; alkylisoquinolinium salt; dialkylmorphonium salt; POE-alkylamine; alkylamine salt; polyamine fatty acid derivatives; amyl alcohol fatty acid derivatives; benzalkonium chloride; and benzethonium chloride.

Examples of the amphoteric surfactants include, but are not limited to, imidazoline-based amphoteric surfactants (for example, 2-undecyl-N,N,N-(hydroxyethylcarboxymethyl)-2-imidazoline sodium salt and 2-cocoyl-2-imidazolinium hydroxide-1-carboxyethyloxydisodium salt); and betaine-based surfactants (for example, 2-heptadecyl-N-carboxymethyl-N-hydroxyethylimidazolinium betaine, lauryldimethylaminoacetic acid betaine, alkylbetaine, amidobetaine, and sulfobetaine).

Examples of lipophilic nonionic surfactants include, but are not limited to, sorbitan fatty acid esters (for example, sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate); glycerin polyglycerin fatty acid (for example, mono cottonseed oil fatty acid glycerin, glyceryl monoerucate, glyceryl sesquioleate, glyceryl monostearate, α,α'-oleic acid pyroglutamic acid glycerin, and monostearic acid glycerin malic acid); propylene glycol fatty acid esters (for example, propylene glycol monostearate); hydrogenated castor oil derivatives; and glycerin alkyl ethers.

Examples of hydrophilic nonionic surfactants include, but are not limited to, POE-sorbitan fatty acid esters (for example, POE-sorbitan monooleate, POE-sorbitan monostearate, POE-sorbitan monooleate, and POE-sorbitan tetraoleate); POE-sorbit fatty acid esters (for example, POE-sorbit monolaurate, POE-sorbit monooleate, POE-sorbit pentaoleate, and POE-sorbit monostearate); POE-glycerin fatty acid esters (for example, POE-monostearates such as POE-glyceryl monostearate, POE-glyceryl monoisostearate, and POE-glyceryl triisostearate); POE-fatty acid esters (for example, POE-distearate, POE-monodioleate, and ethylene glycol distearate); POE-alkyl ethers (for example, POE-lauryl ether, POE-oleyl ether, POE-stearyl ether, POE-behenyl ether, POE-2-octyldodecyl ether, and POE-cholestanol ether); poloxamers (for example, Pluronic); POE/POP-alkyl ethers (for example, POE/POP-cetyl ether, POE/POP-2-decyltetradecyl ether, POE/POP-monobutyl ether, POE/POP-hydrogenated lanolin, and POE/POP-glyceryl ether); tetraPOE/tetraPOP-ethylenediamine condensates (for example, Tetronic); POE-castor oil hydrogenated castor oil derivatives (for example, POE-castor oil, POE-hydrogenated castor oil, POE-hydrogenated castor oil monoisostearate, POE-hydrogenated castor oil triisostearate, POE-hydrogenated castor oil monopyroglutamic acid monoisostearic acid diester, and POE-hydrogenated castor oil maleic acid); POE-beeswax/lanolin derivatives (for example, POE-sorbit beeswax); alkanolamides (for example, coco fatty acid diethanolamide, lauric acid monoethanolamide, and fatty acid isopropanolamide); POE-propylene glycol fatty acid ester; POE-alkylamine; POE-fatty acid amide; sucrose fatty acid ester; alkylethoxydimethylamine oxide; and trioleyl phosphate.

Examples of the humectants include, but are not limited to, polyethylene glycol, propylene glycol, glycerin, 1,3-butylene glycol, xylitol, sorbitol, maltitol, chondroitin sulfate, hyaluronic acid, mucoitin sulfate, caronic acid, atelocollagen, cholesteryl-12-hydroxystearate, sodium lactate, bile salt, dl-pyrrolidone carboxylate, alkylene oxide derivatives, short-chain soluble collagen, triglyceryl (EO) PO adducts, burr rose extract, yarrow extract, and melilot extract.

Examples of natural water-soluble polymers include, but are not limited to, plant-based polymers (for example, gum arabic, gum tragacanth, galactan, guar gum, carob gum, gum karaya, carrageenan, pectin, agar, quince seed (quince), algae colloid (cassava extract), starch (rice, corn, potato, and wheat) and glycyrrhizic acid); microbe-based polymers (for example, xanthan gum, dextran, succinoglycan, and pullulan); and animal-based polymers (for example, collagen, casein, albumin, and gelatin).

Examples of semi-synthetic water-soluble polymers include, but are not limited to, starch-based polymers (for example, carboxymethyl starch and methyl hydroxypropyl starch); cellulose-based polymers (methyl cellulose, ethyl cellulose, methyl hydroxypropyl cellulose, hydroxyethyl cellulose, sodium cellulose sulfate, hydroxypropyl cellulose, carboxymethyl cellulose, sodium carboxymethyl cellulose, crystal cellulose, and cellulose powder); and alginate-based polymers (for example, sodium alginate and propylene glycol alginate).

Examples of synthetic water-soluble polymers include, but are not limited to, vinyl-based polymers (for example, polyvinyl alcohol, polyvinyl methyl ether, polyvinyl pyrrolidone, and carboxyvinyl polymer); polyoxyethylene-based polymers (for example, polyoxyethylene-polyoxypropylene copolymer of polyethylene glycol 20,000, 40,000, or 60,0000); acrylic polymers (for example, sodium polyacrylate, polyethylacrylate, and polyacrylamide); polyethyleneimine; and cationic polymers.

Examples of the thickeners include, but are not limited to, gum arabic, carrageenan, gum karaya, gum tragacanth, carob gum, quince seed (quince), casein, dextrin, gelatin, sodium pectate, sodium alginate, methyl cellulose, ethyl cellulose, CMC, hydroxyethyl cellulose, hydroxypropyl cellulose, PVA, PVM, PVP, sodium polyacrylate, carboxyvinyl polymer, locust bean gum, guar gum, tamarind gum, dialkyldimethylammonium cellulose sulfate, xanthan gum, magnesium aluminum silicate, bentonite, hectorite, Al-Mg silicate (Veegum), laponite, and anhydrous silicic acid.

Examples of the ultraviolet absorbers include, but are not limited to, benzoic acid-based ultraviolet absorbers (for example, para-aminobenzoic acid (hereinafter, abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA ethyl ester); anthranilic acid-based ultraviolet absorbers (for example, homomenthyl-N-acetylanthranilate); salicylic acid-based ultraviolet absorbers (for example, amyl salicylate, menthyl salicylate, homomenthyl salicylate, octyl salicylate, phenyl salicylate, benzyl salicylate, and p-isopropanol phenyl salicylate); cinnamic acid-based ultraviolet absorbers (for example, octyl methoxycinnamate, ethyl-4-isopropyl cinnamate, methyl-2,5-diisopropyl cinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-p-methoxycinnamate(2-ethylhexyl-p-methoxycinnamate), 2-ethyoxyethyl-p-methoxycinnamate, cyclohexyl-p-methoxycinnamate, ethyl-α-cyano-β-phenylcinnamate, 2-ethylhexyl-α-cyano-β-phenylcinnamate, and glyceryl mono-2-ethylhexanoyl-diparamethoxycinnamate); benzophenone-based ultraviolet absorbers (for example, 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone); 3-(4'-methylbenzylidene)-d,l-camphor, 3-benzylidene-d,l-camphor; 2-phenyl-5-methylbenzoxazole; 2,2'-hydroxy-5-methylphenylbenzotriazole; 2-(2'-hydroxy-5' -t-octylphenyl)benzotriazole; 2-(2' -hydroxy-5' -methyl phenylbenzotriazole); dibenzalazine; dianisoylmethane; 4-methoxy-4'-t-butyldibenzoylmethane; 5-(3,3-dimethyl-2-norbomylidene)-3-pentan-2-one, dimorpholinopyridazino; 2-ethylhexyl-2-cyano-3,3-diphenylacrylate; and 2,4-bis-{[4-(2-ethylhexyloxy)-2-hydroxy]-phenyl}-6-(4-methoxyphenyl)-(1,3,5)-triazine.

Examples of metal ion sequestrants include, but are not limited to, 1-hydroxyethane-1,1-diphosphonic acid, 1-hydroxyethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, and trisodium ethylenediamine hydroxyethyl triacetate.

Examples of the lower alcohols include, but are not limited to, ethanol, propanol, isopropanol, isobutyl alcohol, and t-butyl alcohol.

Examples of the polyhydric alcohols include, but are not limited to, dihydric alcohols (for example, ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol); trihydric alcohols (for example, glycerin and trimethylolpropane); tetrahydric alcohols (for example, pentaerythritols such as 1,2,6-hexanetriol); pentahydric alcohols (for example, xylitol); hexahydric alcohols (for example, sorbitol and mannitol); polyhydric alcohol polymers (for example, diethylene glycol, dipropylene glycol, triethylene glycol, polypropylene glycol, tetraethylene glycol, diglycerin, polyethylene glycol, triglycerin, tetraglycerin, and polyglycerin); dihydric alcohol alkyl ethers (for example, ethylene glycol monomethyl ether, ethylene glycol monoethyl ether, ethylene glycol monobutyl ether, ethylene glycol monophenyl ether, ethylene glycol monohexyl ether, ethylene glycol mono 2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether); dihydric alcohol alkyl ethers (for example, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether); dihydric alcohol ether esters (for example, ethylene glycol monomethyl ether acetate, ethylene glycol monoethyl ether acetate, ethylene glycol monobutyl ether acetate, ethylene glycol monophenyl ether acetate, ethylene glycol diadivate, ethylene glycol disuccinate, diethylene glycol monoethyl ether acetate, diethylene glycol monobutyl ether acetate propylene glycol monomethyl ether acetate, propylene glycol monoethyl ether acetate, propylene glycol monopropyl ether acetate and propylene glycol monophenyl ether acetate); glyceryl monoalkyl ethers (for example, chimil alcohol, serachyl alcohol, and batyl alcohol); sugar alcohols (for example, sorbitol, maltitol, maltotriose, mannitol, sucrose, erythritol, glucose, fructose, starch degradation sugars, maltose, xylitose, and starch degradation sugar-reducing alcohols); Glysolid; tetrahydrofurfuryl alcohol; POE-tetrahydrofurfuryl alcohol; POP-butyl ether; POP/POE-butyl ether; tripolyoxypropylene glycerin ether; POP-glycerin ether; POP-glycerin ether phosphate; POP/POE-pentaerythritol ether; and polyglycerin.

Examples of monosaccharides include, but are not limited to, trioses (for example, D-glyceraldehyde and D-dihydroxyacetone); tetroses (for example, D-erythrose, D-erythrulose, D-threose, and erythritol); pentoses (for example, L-arabinose, D-xylose, L-xylose, D-arabinose, D-ribose, D-ribulose, D-xylulose, and L-xylulose); hexoses (for example, D-glucose, D-talose, D-mannose, D-tagatose, D-fructose, D-galactose, L-galactose, D-sorbose, and L-sorbose); heptoses (for example, D-glycero-D-manno-heptose and L-glycero-L-manno-heptose); octoses (for example, D-octulose); deoxy sugars (for example, 2-deoxy-D-ribose, 6-deoxy-L-galactose, and 6-deoxy-L-mannose); amino sugars (for example, D-glucosamine, D-galactosamine, sialic acid, aminouracil, and muramic acid); and uronic acids (for example, D-glucuronic acid, D-mannuronic acid, L-guluronic acid, D-galacturonic acid, and L-iduronic acid).

Examples of the oligosaccharides include, but are not limited to, sucrose, gentianose, umbelliferose, lactose, planteose, isolychnoses, α,α-trehalose, raffinose, lychnoses, umbilicin, and stachyose-verbascoses.

Examples of the polysaccharides include, but are not limited to, cellulose, quince seed, chondroitin sulfate, starch, galactan, dermatan sulfate, glycogen, gum arabic, heparan sulfate, hyaluronic acid, gum tragacanth, keratan sulfate, chondroitin, xanthan gum, mucoitin sulfate, guar gum, dextran, keratosulfate, locust bean gum, succinoglucan, and caronic acid.

Examples of the amino acids include neutral amino acids (for example, threonine and cysteine); and basic amino acids (for example, hydroxylysine). Examples of amino acid derivatives include, but are not limited to, sodium acyl sarcosine (sodium lauroyl sarcosine), acyl glutamate, sodium acyl β-alanine, glutathione, and pyrrolidone carboxylic acid.

Examples of the organic amines include, but are not limited to, monoethanolamine, diethanolamine, triethanolamine, morpholine, triisopropanolamine, 2-amino-2-methyl-1,3-propanediol, and 2-amino-2-methyl-1-propanol.

Examples of the polymer emulsions include, but are not limited to, acrylic resin emulsion, polyacrylic acid ethyl emulsion, acrylic resin solution, polyacrylic alkyl ester emulsion, polyvinyl acetate resin emulsion, and natural rubber latex.

Examples of the pH adjusters include, but are not limited to, buffering agents such as lactic acid-sodium lactate, citric acid-sodium citrate, and succinic acid-sodium succinate.

Examples of the vitamins include, but are not limited to, vitamins A, B1, B2, B6, C, and E, and derivatives thereof, pantothenic acid and derivatives thereof, and biotin.

Examples of the antioxidants include, but are not limited to, tocopherols, dibutylhydroxytoluene, butylhydroxyanisole, and tannic acid esters.

Examples of the antioxidant aids include, but are not limited to, phosphoric acid, citric acid, ascorbic acid, maleic acid, malonic acid, succinic acid, fumaric acid, cephalin, hexametaphosphate, phytic acid, and ethylenediaminetetraacetic acid.

Examples of additional components that can be blended include, but are not limited to, preservatives (ethylparaben, butylparaben, chlorphenesin, and phenoxyethanol); antiphlogistic agents (for example, glycyrrhizic acid derivatives, glycyrrhetinic acid derivatives, salicylic acid derivatives, hinokitiol, zinc oxide, and allantoin); depigmenting agents (for example, placenta extract, *Saxifraga stolonifera* extract, and arbutin); various extracts (for example, cork-tree bark, goldthread rhizome, lithospermum root, Chinese peony root, swertia herb, birch, sage, loquat leaf, ginseng root, aloe, mallow, iris, grape, Job's Tears seed, sponge gourd, lily, saffron, cnidium root, ginger, St. John's wort, restharrow, garlic, chili pepper, aged mikan orange peel, Chinese angelica root, and seaweed); activators (for example, royal jelly, photosensors, and cholesterol derivatives); blood circulation promoters (for example, nonanoic acid vanillylamide, nicotinic acid benzyl ester, nicotinic acid β-butyl ethyl ester, capsaicin, gingerol, cantharidin, ichthammol, tannic acid, α-bomeol, tocopherol nicotinate, inositol hexanicotinate, cyclandelate, cinnarizine, trazodone, acetylcholine, verapamil, cepharanthine, and γ-oryzanol); anti-seborrheic agents (for example, sulfur and thianthol); and anti-inflammatory agents (for example, tranexamic acid, thiotaurine, and hypotaurine).

Metal sequestrants such as disodium edetate, trisodium edetate, sodium citrate, sodium polyphosphate, sodium metaphosphate, gluconic acid, and malic acid; caffeine, tannin, verapamil, tranexamic acid and derivatives thereof; extracts of various herbal medicines such as licorice, Chinese quince, and shinleaf; drugs such as tocopherol acetate, glycyrrhetinic acid, glycyrrhizinic acid, derivatives thereof, and salts thereof; depigmenting agents such as vitamin C, magnesium ascorbyl phosphate, ascorbyl glucoside, arbutin, and kojic acid; amino acids such as arginine and lysine, and derivatives thereof; and saccharides such as fructose, mannose, erythritol, trehalose, and xylitol can also be appropriately blended.

### <Cosmetic product form>

The product form of the cosmetic of the present invention is not particularly limited, and can take on any product form in the category of powder cosmetics. More specifically, examples thereof include, but are not limited to, product forms such as powder foundation, eye shadow, cheek color, body powder, perfume powder, baby powder, pressed powder, deodorant powder, face powder, and loose powder.

From the viewpoint of enabling an improvement in moldability and impact resistance, the cosmetic of the present invention is applied particularly to solid powder cosmetics such as powder foundation, eye shadow, cheek color, and pressed powder.

### <<Method for manufacturing powder cosmetic>>

The cosmetic of the present invention described above can be manufactured by the methods for manufacturing a powder cosmetic described below.

### (First method for manufacturing cosmetic of the present invention)

In the present invention, the first method is
a method comprising:
dispersing the powder component and the oily component described above in a solvent to prepare a slurry;
filling the slurry into a container; and
removing the volatile solvent from the slurry after container filling.

The solvent may be a volatile solvent, or may be a nonvolatile solvent. The volatile solvent may be, for example, a solution containing water as the main dispersion medium and 0 to 30% by mass of a volatile organic solvent that is soluble in water, such as ethyl alcohol, acetone, or isopropyl alcohol, as a secondary dispersion medium. From the viewpoint of improving moldability and impact resistance, it is preferable that the amount of the secondary dispersion medium be adjusted so that the contact angle of the volatile dispersion medium relative to a uniform mixture of all cosmetic components is 125 to 135°. Examples of the nonvolatile solvent include, but are not limited to, nonvolatile silicone-based solvents.

Examples of the method of preparing the slurry include a method of adding the powder component and the oily component preliminarily dry-mixed/pulverized with a Henschel^{®} mixer or a pulperizer to a dispersion solvent and then mixing/dispersing the mixture with a dispersion mixer, a homogenizer, a planetary mixer, a Combimix^{®}, an Agi Homo mixer, or a twin-screw kneader. When the oily component comprises an oil that is solid or paste-like at 25 °C, it is preferable that the oily component be melted by heating and then dry-mixed with the powder component.

When preparing the slurry, the quantity ratio (mass ratio) of the powder component to the oily component depends on the oily component and the powder component used, and may be at a ratio of powder component/oily component = 60/40 to 99.5/0.5. The amount of dispersion solvent used at this time cannot be specified as the amount depends on the polarity and specific gravity of the dispersion solvent used. However, the amount used is generally about half to twice the amount of the cosmetic component as it is important to ensure sufficient fluidity during filling and molding.

The slurry prepared as described above can be suitably filled into a container such as an inner tray made of a metal or a resin by injection filling.

The powder cosmetic of the present invention can be obtained by removing the solvent from the slurry after container filling.

The method of removing the solvent may be carried out, for example, by suction press molding, and may further comprise drying with an appropriate dryer thereafter.

The first method may also be referred to as a "wet manufacturing method".

### (Second method for manufacturing cosmetic of the present invention)

In the present invention, the second method is
a method comprising:
dispersing the powder component and the oily component described above in a solvent to prepare a slurry; and
drying the slurry to obtain a dry powder.

Since the method for preparing the slurry and the details of the solvent may be the same as the "first method" described above, the description therefor is omitted. In addition, the "slurry preparation step" and "volatile solvent" (particularly when the solvent is used as a volatile solvent) described in Japanese Unexamined Patent Publication (Kokai) No. 2007-55990 may be appropriately adopted.

The prepared slurry can be dried to obtain a dry powder.

When drying the slurry, for example, a drying apparatus that dropletizes the slurry by a mechanical shearing force and blows a dry gas at the fine droplets to dry the slurry may be used. The drying apparatus may be a drying apparatus comprising a hollow housing, a shearing means for shearing the slurry with a shearing member provided in the housing into minute droplets, a supplying means for supplying the slurry to the shearing member inside the housing, a blowing means for blowing a dry gas into the housing and supplying and contacting the slurry made into minute droplets by the shearing means with the dry gas, and a collecting means for collecting the dry powder produced by drying the slurry. For details of slurry drying and the drying apparatus used, for example, Japanese Unexamined Patent Publication No. 2007-55990 may be appropriately referenced.

The second method of the present invention may further comprise filling the obtained dry powder in a container and solidifying by dry molding.

The second method may also be referred to as a "W&D manufacturing method".

### EXAMPLES

The present invention will be further described in detail with reference to the Examples below. However, the present invention is not limited thereto. Note that the amount of each component is indicated in % by mass unless otherwise specified.

### «Examples 1 to 3 and Comparative Examples 1 to 4»

Manufactured by a W&D manufacturing method:
A powder component and an oily component described in the formulations shown in Table 1 below were mixed and then mixed in ethyl alcohol with a dispersion mixer. The slurry viscosity was adjusted to about 4000 mPa·s and mixing/dispersing was carried out using a twin-screw extruder. A powder slurry was obtained thereby.

The resulting powder slurry was dried in the form of minute droplets using a stirring drying apparatus (spin flash dryer, manufactured by APV Nordic Anhydro) to obtain a dry powder.

The obtained dry powder was filled into an inner tray container made of resin and subjected to dry press molding by a known method to obtain a solid powder cosmetic for each of Examples 1 to 3 and Comparative Examples 1 to 4.

### «Example 4»

Manufactured by a wet manufacturing method:
A powder component and an oily component described in the formulations shown in Table 1 below were mixed using a Henschel mixer and further pulverized using a pulperizer to obtain a uniform mixture. Water (= volatile dispersion medium) in an equal amount to the mixture was added thereto and mixed using a dispersion mixer to obtain a slurry. The slurry was filled in an inner tray, the solvent was removed by suction, and then drying was carried out to obtain a solid powder cosmetic used in Example 4.

### «Evaluation of overall usability»

The powder solid cosmetic of each of the Examples and Comparative Examples obtained above was evaluated for overall usability (soft feel, silky touch, and powderiness) when applied to the skin, based on the following criteria. The results are shown in Table 2.

### (Soft feel)

"A": Sample loosened well when the surface was rubbed with a sponge, and had a very light, moist, and soft touch when applied;
"B": Sample loosened well when the surface was rubbed with a sponge, and had a moist touch when applied;
"C": Sample barely loosened when the surface was rubbed with a sponge, and had a dry touch when applied;
"D": Sample barely loosened when the surface was rubbed with a sponge, and tended to catch the skin and had a heavy touch when applied.

### (Silky touch)

"A": Sample loosened well when the surface was rubbed with a sponge, had a moderately moist feeling when applied, and expanded/spread very well on the skin;
"B": Sample loosened well when the surface was rubbed with a sponge, and expanded/spread well on the skin;
"C": Sample barely loosened when the surface was rubbed with a sponge, tended to catch when applied, and felt slightly heavy;
"D": Sample barely loosened when the surface was rubbed with a sponge, tended to catch when applied, felt slightly heavy, and did not spread well on the skin.

### (Powderiness)

"A": Sample loosened well when the surface was rubbed with a sponge, expanded/spread and fit the skin well when applied thereon, and was absorbed quickly;
"B": Sample loosened well when the surface was rubbed with a sponge, fit well when applied, expanded/spread well when applied, and blended in;
"C": Sample barely loosened when the surface was rubbed with a sponge, powder remained on the skin when applied, and a slightly dry sensation was felt;
"D": Sample barely loosened when the surface was rubbed with a sponge, and powder remained on the skin when applied, did not absorb well into the skin, and left behind a dry sensation.

### <<Evaluation of moldability>>

The powder solid cosmetic of each of the Examples and Comparative Examples obtained above was evaluated for moldability. More specifically, the same resin-like inner tray was set in a mold, 10 g of a sample was added, and a pressure of 2.0 MPa was applied for molding. The moldability was evaluated based on the following criteria. The results are shown in Table 2.
"A": Sample could be molded into a resin-like inner tray by pressing at a constant pressure;
"B": Sample could be molded into a resin-like inner tray by pressing at a constant pressure but was brittle;
"C": Sample did not solidify and cannot be molded even when pressed at a constant pressure.

### «Evaluation of impact resistance»

The powder solid cosmetic of each of the Examples and Comparative Examples obtained above was set in a compact container for cosmetics and dropped onto a metal plate from a height of 30 cm with the cosmetic surface facing downward. The number of drops until cracking was examined. Impact resistance was evaluated based on the following criteria. The results are shown in Table 2.
"A": cracked at 8 times or more;
"B": cracked at 6 or 7 times;
"C": cracked at 3 to 5 times;
"D": cracked within twice (also corresponds to when molding was not possible).

**[Table 1]**

| (Table 1: Formulation) | | | | | | | | | |
|---|---|---|---|---|---|---|---|---|---|
| Component | | Example 1 | Example 2 | Example 3 | Comparative Example 1 | Comparative Example 2 | Comparative Example 3 | Example 4 | Comparative Example 4 |
| Powder component | Mica | 49.42 | 49.42 | 49.42 | 49.42 | 49.42 | 49.42 | 49.42 | 49.42 |
| | Boron nitride | 7 | 7 | 7 | 7 | 7 | 7 | 7 | 7 |
| | Titanium oxide | 14 | 14 | 14 | 14 | 14 | 14 | 14 | 14 |
| | Zinc oxide | 4 | 4 | 4 | 4 | 4 | 4 | 4 | 4 |
| | Component (A): hollow silica particles (average particle size: 4 µm) | 7 | 6 | 5 | 11 | - | - | 5 | - |
| | Component (B): solid silica (average particle size: 5 µm) | 4 | - | 2 | - | 11 | - | 2 | - |
| | Component (C): porous silica (average particle size: 10 m) | - | 5 | 4 | - | - | 11 | 4 | - |
| | Polymethylmethacrylate (PMMA) | - | - | - | - | - | - | - | 1.5 |
| | (Vinyl dimethicone/dimethicone) crosspolymer | - | - | - | - | - | - | - | 4 |
| | Urethane powder | - | - | - | - | - | - | - | 2 |
| | Hydrophobized iron oxide (red) | 0.08 | 0.08 | 0.08 | 0.08 | 0.08 | 0.08 | 0.08 | 0.08 |
| | Hydrophobized iron oxide (yellow) | 1.44 | 1.44 | 1.44 | 1.44 | 1.44 | 1.44 | 1.44 | 1.44 |
| | Hydrophobized iron oxide (black) | 0.14 | 0.14 | 0.14 | 0.14 | 0.14 | 0.14 | 0.14 | 0.14 |
| | Chlorphenesin | 0.2 | 0.2 | 0.2 | 0.2 | 0.2 | 0.2 | 0.2 | 0.2 |
| Oily component | Dimethicone-6 CS | 2.7 | 2.7 | 2.7 | 2.7 | 2.7 | 2.7 | 2.7 | 2.7 |
| | Glyceryl tri(caprylate/caprate) | 2.5 | 2.5 | 2.5 | 2.5 | 2.5 | 2.5 | 2.5 | 2.5 |
| | Ethylhexyl methoxycinnamate | 5.0 | 5.0 | 5.0 | 5.0 | 5.0 | 5.0 | 5.0 | 5.0 |
| | (Stearoxymethicone/dimethicone) copolymer | 1.8 | 1.8 | 1.8 | 1.8 | 1.8 | 1.8 | 1.8 | 1.8 |
| | Phenoxyethanol | 0.5 | 0.5 | 0.5 | 0.5 | 0.5 | 0.5 | 0.5 | 0.5 |
| | Ethylhexylglycerin | 0.2 | 0.2 | 0.2 | 0.2 | 0.2 | 0.2 | 0.2 | 0.2 |
| | Tocopherol | 0.02 | 0.02 | 0.02 | 0.02 | 0.02 | 0.02 | 0.02 | 0.02 |
| Total (% by mass) | | 100 | 100 | 100 | 100 | 100 | 100 | 100 | 100 |
| Manufacturing method | | W&D manufacturing method | | | | | | Wet manufacturing method | W&D manufacturing method |

**[Table 2]**

| (Table 2: Evaluation results) | | | | | | | | |
|---|---|---|---|---|---|---|---|---|
| Evaluation result | Example 1 | Example 2 | Example 3 | Comparative Example 1 | Comparative Example 2 | Comparative Example 3 | Example 4 | Comparative Example 4 |
| Soft touch | B | B | A | A | C | D | A | A |
| Silky touch | C | A | B | D | C | A | B | B |
| No "crumbliness/powderiness" | B | B | A | D | C | D | A | A |
| Moldability | B | B | A | C | B | D | B | A |
| Impact resistance/Number of drops | C/5 times | B/6 times | A/8 times | D/2 times | C/4 times | D/not moldable | A/10 times | B/7 times |

As is clear from Table 2, the cosmetics of Examples 1 to 4 were found to have improved overall usability and improved moldability and impact resistance, compared to the cosmetics of Comparative Examples 1 to 4.

## Claims

1. A cosmetic, which is a powder cosmetic comprising 80 to 99% by mass of a powder component and 1.0 to 20% by mass of an oily component, wherein
the powder component comprises a component (A) below and at least one of a component (B) and a component (C) below:
component (A): hollow silica particles, having an average particle size of 1 to 10 µm,
component (B): solid silica particles, having an average particle size of 2 to 7 µm, and
component (C): porous silica particles, having an average particle size of 1 to 30 µm.

2. The cosmetic according to claim 1, wherein the powder component comprises the component (A), the component (B), and the component (C).

3. The cosmetic according to claim 1 or 2, which is a solid powder cosmetic.

4. A method for manufacturing the cosmetic according to any one of claims 1 to 3, comprising:
dispersing the powder component and the oily component in a solvent to prepare a slurry;
filling the slurry into a container; and
removing the solvent from the slurry after container filling.

5. A method for manufacturing the cosmetic according to any one of claims 1 to 3, comprising:
dispersing the powder component and the oily component in a solvent to prepare a slurry; and
drying the slurry to obtain a dry powder.
